(19) Europäisches Patentamt
European Patent Office
Office européen des brevets

(11) Numéro de publication: **0 139 588**
**B1**

(12) **FASCICULE DE BREVET EUROPEEN**

(45) Date de publication du fascicule du brevet: **01.07.87**

(21) Numéro de dépôt: **84402047.9**

(22) Date de dépôt: **11.10.84**

(51) Int. Cl.⁴: **C 08 F 220/56,** A 61 K 7/06 // (C08F220/56, 220:58, 220:60, 220:34)

(54) **Tétrapolymères ampholytes cationiques, leur application dans le traitement des fibres kératiniques et les compositions les renfermant.**

(30) Priorité: **19.10.83 FR 8316621**

(43) Date de publication de la demande:
**02.05.85 Bulletin 85/18**

(45) Mention de la délivrance du brevet:
**01.07.87 Bulletin 87/27**

(84) Etats contractants désignés:
**BE CH DE GB IT LI NL**

(56) Documents cités:
**EP-A-0 045 720**

(73) Titulaire: **SOCIETE FRANCAISE HOECHST**
**Société anonyme dite:**
**3, avenue du Général de Gaulle**
**F-92800 Puteaux (FR)**

(72) Inventeur: **Cabestany, Jean**
**127 Boulevard Maxime Gorki**
**F-93240 Stains (FR)**
Inventeur: **Siegel, Daniel**
**4, rue des Sablières**
**F-78360 Montesson (FR)**
Inventeur: **Righetti, Roland**
**7 Square Copernic**
**F-78150 Le Chesnay (FR)**

(74) Mandataire: **Rinuy, Santarelli**
**14, avenue de la Grande Armée**
**F-75017 Paris (FR)**

**EP 0 139 588 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**Description**

La présente invention concerne des tétrapolymères ampholytes cationiques, leur application dans le traitement des fibres kératiniques et les compositions les renfermant.

Il est connu d'employer dans des compositions cosmétiques destinées au traitement des fibres kératiniques, des polymères anioniques, des polymères cationiques, des ionomères ou leurs mélanges.

Malgré leur intérêt, ces compositions ne permettant pas d'obtenir simultanément et au moindre prix toutes les qualités souhaitées par la clientèle d'aujourd'hui.

En effet, et plus particulièrement dans le traitement du cheveu, l'utilisateur souhaite disposer, au moindre prix et pour un usage courant, de compositions performantes, stables dans le temps, compatibles avec les tensio-actifs anioniques communément utilisés dans cette application, tels que le lauryléthersulfate de sodium, donnant des cheveux brillants, nerveux, non-poisseux, non-électriques, doux au toucher, faciles à coiffer, de bonne tenue et conférant à la chevelure du volume.

Or, la Demanderesse a découvert des compositions destinées au traitement des fibres kératiniques repondant aux souhaits et à la demande de la clientèle.

Ce but est atteint grâce à l'emploi dans les compositions selon l'invention de nouveaux tétrapolymères ampholytes cationiques hydrosolubles.

Ces nouveaux tétrapolymères ampholytes cationiques hydrosolubles, objet de la présente invention, peuvent être représentés, d'une façon très schématique par la formule générale suivante:

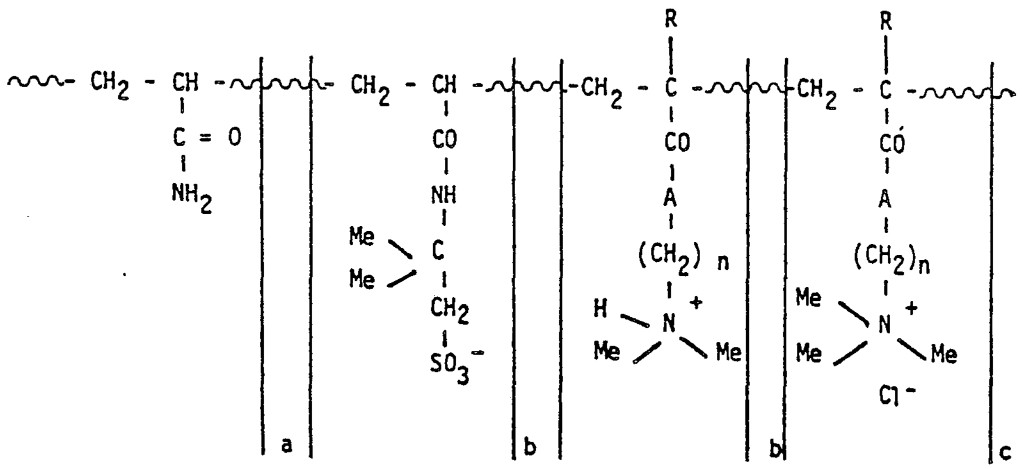

dans laquelle A représente un atome d'oxygène ou un groupement NH, R représente un atome d'hydrogène ou un groupement méthyle lorsque A représente un groupement NH et uniquement un atome d'hydrogène lorsque A représente un atome d'oxygène, *n* est égal à 2 lorsque A représente un atome d'oxygène et *n* est égal à 3 lorsque A représente un groupement NH, les lettres *a, b, c* représentent le % molaire de chaque monomère utilisé et elles répondent aux relations suivantes:

$$a + 2b + c = 100$$

$$30 < a < 75$$

$$10 < b < 25$$

$$5 < c < 20$$

L'invention a plus particulièrement pour objet les tétrapolymères tels que définis ci-dessus caractérisés en outre en ce q'uils résultent de la copolymérisation selon des moyens connus en soi des quatre monomères connus appartenant aux quatre classes de monomères suivantes et avec des pourcentages molaires:

1 — a % d'acrylamide, AAM;

2 — b % d'acide acrylamido-2 méthyl-2 propanesulfonique, AMPS;

3 — b % d'un monomère basique choisi parmi les trois monomères suivants:
- — acrylate de diméthylaminoéthyle, ADAME;
- — N-(diméthylamino-3 propyl-1) acrylamide, DMAPA;
- — N-(diméthylamino-3 propyl-1) méthacrylamide, DMAPMA;

4 — c % d'un monomère cationique choisi parmi les quatre monomères suivants:
- — chlorure de N,N,N-triméthylacrylamido-3 propanaminium, APTAC;
- — chlorure de N,N,N-triméthylméthacrylamido-3 propanaminium, MAPTAC;

— chlorure de N,N,N-triméthylacryloyloxy-2 éthanaminium, CMA;

— chlorhydrate d'acrylate de diméthylaminoéthyle, CHA;

Les lettres *a, b* et *c* ayant la signification ci-dessus.

Ce qui signifie en d'autres termes, que dans les tétrapolymères ampholytes cationiques selon l'invention, le pourcentage molaire d'acrylamide est compris entre 30 et 75%, les pourcentages molaires d'acide acrylamido-2 méthyl 2 propanesulfonique et de monomère basique sont égaux et sont compris entre 10 et 25% et le pourcentage molaire en monomère cationique est compris entre 5 et 20%.

L'invention a tout spécialement pour objet:

— un tétrapolymère répondant à la définition générale ci-dessus caractérisé en outre en ce qu'il contient, en proportions molaires, 75% d'acrylamide, 10% d'acide acrylamido-2 méthyl-2 propanesulfonique, 10% de N-(diméthylamino-3 propyl-1) méthacrylamide et 5% de chlorure de N,N,N-triméthylméthacrylamido-3 propanaminium; et

— un tétrapolymère répondant à la définition générale ci-dessus caractérisé en outre en ce qu'il contient, en proportions molaires, 40% d'acrylamide, 20% d'acide acrylamido-2 méthyl-2 propanesulfonique, 20% de N-(diméthylamino-3 propyl-1) méthacrylamide et 20% de chlorure de N,N,N-triméthylméthacrylamido-3 propanaminium.

Les rapports de réactivité, $r_1$ et $r_2$, de ces monomères acide, basiques ou aminiums avec l'acrylamide sont favourables à une copolymérisation homogène. Pour le couple AAM—AMPS, $r_1 = 1$ et $r_2 = 0,52$ (C. L. McCORMICK et collaborateurs, J. POLYMER Sci. C, 1982, *20*, 817—838), pour le couple AAM—ADAME salifié ou quaternisé, $r_1 = 0,7$—$0,8$ et $r_2 = 0,55$—$0,66$ (brevet des Etats-Unis d'Amérique No. 4 396 752), pour le couple AAM—DMAPMA, $r_1 = 0,47$ et $r_2 = 0,98$ et pour le couple AAM—MAPTAC, $r_1 = 0,51$ et $r_2 = 1,08$ (déterminés selon la méthode de T. KELEN et collaborateurs, J. POLYMER Sci. C, 1977, *15*, 3047—3074).

Les tétrapolymères selon la présente invention sont aisément obtenus à partir des monomères précédemment cités, par copolymérisation radicalaire en phase aqueuse homogène désoxygénée, réalisé en atmosphère inerte et à un pH légèrement acide, généralement compris entre 4 et 5 de manière à éviter tout risque d'hydrolyse des fonctions esters éventuellement présentes.

La concentration pondérale initiale en monomères, Co, peut varier de 10 à 40% et les initiateurs classiques hydrosolubles agissant par homolyse thermique, tels que le persulfate d'ammonium peuvent être employés.

La copolymérisation est effectuée à chaud, généralement vers 60—80°C, puis lorsqu'elle est terminée, on neutralise, si nécessaire à pH = 6,7 la solution aqueuse réactionnelle préalablement refroidie à la température ambiante, avec de l'ammoniaque diluée, et, enfin, la solution obtenue est éventuellement protégée par addition de bactéricides et de fongicides connus à des concentrations habituellement utilisées en cosmétologie.

Le tétrapolymère ainsi obtenu en solution aqueuse présente une viscosité intrinsèque déterminée à 20°C en solution molaire de chlorure de sodium, comprise entre 1 et 5 dl/g. Certes, en modifiant la Co et le taux d'amorceur de la copolymérisation, on peut obtenir à la demande, soit une viscosité intrinsèque plus élevée, soit une viscosité intrinsèque plus faible, mais pour qu'une solution aqueuse du tétrapolymère selon l'invention soit utile pour le traitement des fibre kératiniques, il est nécessaire qu'elle soit suffisamment visqueuse pour rester sur les fibres et suffisamment fluide pour mouiller efficacement les fibres.

L'expérience montre que la quantité de tétrapolymère selon l'invention à introduire dans les compositions ou formulations selon l'invention dépend es résultats particuliers que l'on souhaite atteindre. En général, cette quantité doit former une proportion de l'ordre d'environ 0,1 à 5 % en poids exprimés en matières sèches par rapport au poids total de la composition aqueuse.

Quoique les tétrapolymères ampholytes cationiques hydrosolubles de la présente invention constituent les ingrédients actifs essentiels des compositions conformes à la présente invention, ces compositions peuvent également contenir d'autres ingrédients qui peuvent, notamment, améliorer les propriétés organoleptiques ou faciliter l'application.

Ainsi, il convient également, dans le cadre de la présente invention, d'incorporer dans les compositions selon l'invention, un ou plusieurs constituants habituels ou connus de telles compositions comme par exemple des parfums, des colorants pouvant avoir pour fonction de colorer la composition elle-même ou les fibres kératiniques, des agents conservateurs, des agents séquestrants, des agents épaississants, des agents adoucissants, des synergistes de mousse, des stabilisateurs de mousse, de filtres solaires, des agents peptisants ainsi que des agents tensioactifs anioniques, cationiques, amphotères, non-ioniques ou leurs mélanges.

Le support ou véhicule associé aux compositions selon la présente invention est un véhicule ou support aqueux. Ces compositions peuvent prendre n'importe quelle forme: solutions, émulsions ou gels aqueux, etc.

Dans le cadre de la présente invention, le terme "support ou véhicule aqueux" implique tous les cas où l'eau est pratiquement le seul matériau constitutif de ce véhicule, ainsi que ceux dans lesquels l'eau est associée à des proportions relativement importantes à d'autres substances, telles que solvants, agents épaississants, tensioactifs, etc.

Les compositions selon la présente invention peuvent être appliquées au traitement des fibres

kératiniques de n'importe quelle manière convenable. Par exemple, pour les soins du cheveu, une méthode habituelle consiste en l'application d'une composition selon l'invention, décrite dans les exemples, pendant quelques minutes, à des cheveux fraîchement shampouinés, puis à un rinçage du cheveu à l'eau préalablement au peignage.

La quantité de composition selon l'invention appliquée aux fibres kératiniques peut varier dans de grandes proportions selon les désirs de l'utilisateur, mais en général, elle ne doit pas être inférieure à 1% du poids des fibres kératiniques à traiter, et ne doit pas excéder 20% de ce poids.

L'efficacité des tétrapolymères selon l'invention dans le traitement des fibres kératiniques se détermine facilement par quelques essais simples réalisés à partir d'une composition les renfermant.

Parmi ces essais, certains sont effectués sur des mèches de cheveux préalablement shampouinés avec un shampooing commercial, puis traitées humides avec une composition selon l'invention, on observe ensuite visuellement les effets obtenus sur les cheveux humides, puis séchés, tant au niveau de leur douceur, brillance, souplesse, tenue, propriété non-électrique, facilité à être coiffés, que du volume de la coiffure obtenue.

D'autres essais permettent de déterminer les propriétés moussantes des compositions selon l'invention avec le test décrit dans la norme ASTM D 1173—53.

Enfin, la stabilité dans le temps, à la température ambiante de solutions aqueuses contenant 0,5% en poids de tétrapolymère selon l'invention a été étudiée pa suivi de la viscosité Brookfield de ces solutions conservées à la lumière du jour à 20°C, pendant 60 jours, en présence de 0,07% en poids d'un mélange de bactéricides et de fongicides connus et couramment utilisés en cosmétologie. On n'observe pas de variations significatives de la viscosité Brookfield de ces solutions qui restent par ailleurs parfaitement limpides.

D'autres avantages et caractéristiques de la présente invention apparaîtront à la lecture de la description suivante et des exemples données à titre illustratif et nullement limitatif.

Exemple 1

Préparation d'un tétrapolymère ampholyte cationique, AAM—AMPS—DMAPMA—MAPTAC, 40/20/20/20 en proportions molaires.

Dans un réacteur, on chauffe de 20°C à 80°C, à la vitesse de 1 à 1,5°C par minute, sous atmosphère d'azote, une solution aqueuse parfaitement désoxygénée contenant:

— 29,39 g (0,142 mole) d'AMPS
— 24,14 g (0,142 mole) de DMAPMA
— 20,16 g (0,284 mole) d'AAM
— 31,31 g (0,142 mole) de MAPTAC
— 0,787 g (3,45 mmoles) de persulfate d'ammonium
— 595 g d'eau.

L'initiation se produit vers 60°C et lorsque la température de la solution atteint 80°C, on la maintient 1 heure à cette température, puis on introduit à 80°C, 0,262 g (1,5 mmole) de persulfate d'ammonium en solution dans 5 g d'eau et l'on poursuit le chauffage 1 heure supplémentaire à 80°C et ensuite 30 minutes à 85—90°C. Le milieu est ensuite refroidi à la température ambiante, puis on ajuste le pH de la solution à 6,5—7,0 avec de l'ammoniaque diluée à 20%.

On obtient ainsi une solution aqueuse possédant une viscosité Brookfield, déterminée à 20°C, à 20 tours par minute avec l'axe 2, de 0,36 Pa.s et contenant pondéralement 15,5% d'un tétrapolymère ampholyte cationique: AAM—AMPS—DMAPMA—MAPTAC, 40/20/20/20 en proportions molaires et de viscosité intrinsèque, déterminée à 20°C en solution molaire de chlorure de sodium, de 1,01 dl/g.

Exemples 2—6

En opérant selon l'exemple 1, mais au départ de proportions molaires des monomères données dans le tableau I, on obtient des solutions aqueuses contenant des tétrapolymères hydrosolubles ampholytes cationiques à la concentration MA exprimée en grammes pour 100 g et déterminée par séchage d'une prise d'essai de 1 g à 140°C pendant 1 heure. Ces solutions aqueuses présentent une viscosité Brookfield exprimée en millipascals-seconde et déterminée à 20°C avec un viscosimètre Brookfield équipé de l'axe 2 tournant à 20 tours par minute, indiquée dans le tableau V. Les viscosités intrinsèques, [η], des tétrapolymères obtenus sont également données dans le tableau I; ces viscosités ont été déterminées à 25°C en solution molaire de chlorure de sodium.

Le taux en monomères résiduels a été déterminé par dosage classique des liaisons éthyléniques résiduelles, il est exprimé en grammes pour 100 g de solution.

TABLEAU I

| N° | Composition en % molaires | | | | MA % | Viscosité Brookfield m Pa.s | Viscosité intrinsèque dl/g | Taux en monomères résiduels |
|---|---|---|---|---|---|---|---|---|
| | AAM | AMPS | DMAPMA | MAPTAC | | | | |
| 2 | 75 | 10 | 10 | 5 | 15,4 | 2550 | 1,53 | 0,08 |
| 3 | 70 | 10 | 10 | 10 | 15,3 | 2075 | 1,60 | 0,09 |
| | | | | | | | | |
| | AAM | AMPS | ADAME | MAPTAC | | | | |
| 4 | 70 | 10 | 10 | 10 | 15,5 | 890 | 1,25 | 0,25 |
| 5 | 70 | 10 | 10 | 10 | 10,5 | 38250 | 4,15 | |
| | | | | | | | | |
| | AAM | AMPS | ADAME | CHA | | | | |
| 6 | 60 | 10 | 10 | 20 | 15,5 | 2775 | 1,76 | 0,17 |

Exemples 7—15

A — Préparation de compositions pour le traitement du cheveu

A partir de solutions aqueuses de tétrapolymères hydrosolubles ampholytes cationiques décrites dans les exemples 1—6, on prépare des compositions aqueuses pour le traitement du cheveu numérotées de 7 à 14.

Les pourcentages pondéraux en matières actives sèches des divers ingrédients de ces compositions sont mentionnés dans le tableau II.

La compositions n° 15 est une composition témoin.

TABLEAU II

| Ingrédients / Exemples | 7 | 8 | 9 | 10 | 11 | 12 | 13 | 14 | 15 |
|---|---|---|---|---|---|---|---|---|---|
| Tétrapolymère selon ex. 1 | 2,82 | 2,75 | | | | | | | |
| Tétrapolymère selon ex. 2 | | | 2,79 | 2,77 | | | | | |
| Tétrapolymère selon ex. 3 | | | | | 2,78 | | | | |
| Tétrapolymère selon ex. 4 | | | | | | 2,85 | | | |
| Tétrapolymère selon ex. 5 | | | | | | | 4,06 | | |
| Tétrapolymère selon ex. 6 | | | | | | | | 2,85 | |
| LESA | 30 | 30 | 30 | 30 | 30 | 30 | 30 | 30 | 30 |
| Formaldéhyde à 37% | 0,2 | 0,2 | 0,2 | 0,2 | 0,2 | 0,2 | 0,2 | 0,2 | 0,2 |
| Eau q.s. pour 100 g. | 66,98 | 67,05 | 67,01 | 67,03 | 67,02 | 66,95 | 65,74 | 66,95 | 69,80 |
| Poids total | 100,00 | 100,00 | 100,00 | 100,00 | 100,00 | 100,00 | 100,00 | 100,00 | 100,00 |

LESA: lauryléthersulfate de sodium.

B — Effets des compositions sur le cheveu mouillé préalablement shampouiné, puis sur le cheveu sec

Les effets des compositions selon l'invention sur le cheveu mouillé préalablement shampouiné ont été déterminées par examen visuel et en fonction des effets observés on a noté les compositions de 1 à 5 selon le code suivant:

| | |
|---|---|
| excellent | 5 |
| bien | 4 |
| moyen | 3 |
| médiocre | 2 |
| mauvais | 1 |

Les résultats obtenus sont mentionnés dans le tableau III.

De même, on a testé les compositions sur le cheveu une fois séché et on a consigné les observations visuelles de ces effets dans le tableau IV.

Effet de la composition sur cheveu mouillé.

TABLEAU III

| Tests \ N° des formules | 8 | 10 | 11 | 12 | 13 | 14 | 15 |
|---|---|---|---|---|---|---|---|
| Facilité du peignage | | | | | | | |
| a) après traitement | 4 | 4 | 2 | 3 | 5 | 4 | 2 |
| b) après a puis rinçage | 3 | 4 | 3 | 3 | 5 | 3 | 2 |
| Douceur du cheveu | | | | | | | |
| après b | 3 | 3 | 3 | 3 | 4 | 3 | 4 |
| Totaux | 10 | 11 | 8 | 9 | 14 | 10 | 8 |

TABLEAU IV

| Tests \ N° des formules | 7 | 8 | 9 | 10 | 11 | 12 | 13 | 14 | 15 |
|---|---|---|---|---|---|---|---|---|---|
| Facilité du peignage | 4 | 4 | 4 | 4 | 4 | 5 | 4 | 3 | 3 |
| Douceur | 4 | 4 | 4 | 4 | 4 | 4 | 4 | 4 | 3 |
| Gonflant | 4 | 3 | 4 | 4 | 4 | 3 | 4 | 4 | 3 |
| Brillance | 4 | 4 | 4 | 4 | 3 | 4 | 5 | 5 | 3 |
| Propriété non-électrique | 4 | 4 | 4 | 4 | 2 | 2 | 5 | 2 | 3 |
| Mise en forme | 3 | 3 | 3 | 3 | 4 | 5 | 5 | 4 | 4 |
| Totaux | 23 | 22 | 23 | 23 | 21 | 23 | 27 | 22 | 19 |

C — Déterminations du pouvoir moussant des compositions

Le pouvoir moussant de certaines compositions a été déterminé à 40°C, selon la norme ASTM D 1173—53, après dilution des compositions à 3% en poids dans de l'eau de ville. Le tableau V donne les hauteurs de mousse exprimées en centimètres en fonction du temps exprimé en minutes des compositions testées.

TABLEAU V

| N° des formules | t = 0 | t = 1 min. | t = 5 min. |
|---|---|---|---|
| 12 | 24 | 22,5 | 22,5 |
| 14 | 22 | 20,5 | 20,5 |
| 15 | 21 | 20 | 19 |

**Revendications**

1. Tétrapolymères ampholytes cationiques hydrosolubles répondant à la formule générale suivante:

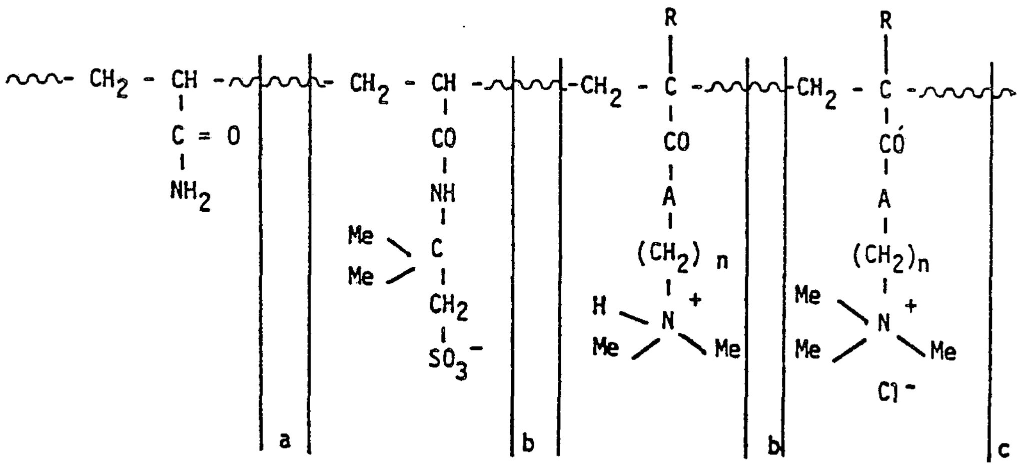

dans laquelle A représente un atome d'oxygène ou un groupement NH, R représente un atome d'hydrogène ou un groupement méthyle lorsque A représente un groupement NH et uniquement un atome d'hydrogène lorsque A représente un atome d'oxygène, *n* est égal à 2 lorsque A représente un atome d'oxygène et *n* est égal à 3 lorsque A représente un groupement NH, les lettres *a, b, c* représentent le % molaire de chaque monomère utilisé et elles répondent aux relations suivantes:

$$a + 2b + c = 100$$

$$30 < a < 75$$

$$10 < b < 25$$

$$5 < c < 20$$

2. Tétrapolymères ampholytes cationiques hydrosolubles delon la revendication 1, caractérisés en outre par le fait qu'ils sont issus de la copolymérisation de:

a moles d'acrylamide, AAM;

b moles d'acide acrylamido-2 méthyl-2 propanesulfonique, AMPS;

b moles d'un monomère basique choisi parmi les trois monomères suivants:

acrylate de diméthylaminoéthyle, ADAME;

N-(diméthylamino-3 propyl-1) acrylamide, DMAPA;

N-(diméthylamino-3 propyl-1)méthacrylamide, DMAPMA;

c moles d'un monomère cationique choisi parmi les quatre monomères suivants:

chlorure de N,N,N-triméthylacrylamido-3 propanaminium, APTAC;

chlorure de N,N,N-triméthylméthacrylamido-3 propanaminium, MAPTAC;

chlorure de N,N,N-triméthylacryloyloxy-2 éthanaminium, CMA;

chlorhydrate d'acrylate de diméthylaminoéthyle, CHA;

avec les relations suivantes:

$$a + 2b + c = 100$$

$$30 < a < 75$$

$$10 < b < 25$$

$$5 < c < 20$$

3. Tétrapolymère selon la revendication 1 ou 2, caractérisé par le fait qu'il contient, en proportions molaires, 75% d'acrylamide, 10% d'acide acrylamido-2 méthyl-2 propanesulfonique, 10% de N-(diméthylamino-3 propyl-1) méthacrylamide et 5% de chlorure de N,N,N-triméthylméthacrylamido-3 propanaminium.

4. Tétrapolymère selon la revendication 1 ou 2, caractérisé par le fait qu'il contient en proportions molaires, 40% d'acrylamide, 20% d'acide acrylamido-2 méthyl-2 propanesulfonique, 20% de N-(diméthylamino-3 propyl-1) méthacrylamide et 20% de chlorure de N,N,N-triméthylméthacrylamido-3 propanaminium.

5. Application des tétrapolymères selon l'une quelconque des revendications 1 à 4 au traitement des fibres kératiniques.

6. Composition aqueuses destinées au traitement des fibres kératiniques, caractérisées par le fait qu'elles contiennent comme matières actives principales un tétrapolymère selon l'une quelconque des revendications 1 à 4.

7. Compositions aqueuses destinées au soin du cheveu selon la revendication 6, caractérisées par le fait qu'elles contiennent pondéralement de 0,1 à 5% exprimés en matières sèches d'un tétrapolymère selon l'une quelconque des revendications 1 à 4.

**Claims**

1. Water-soluble cationic ampholytic tetrapolymers of the following general formula:

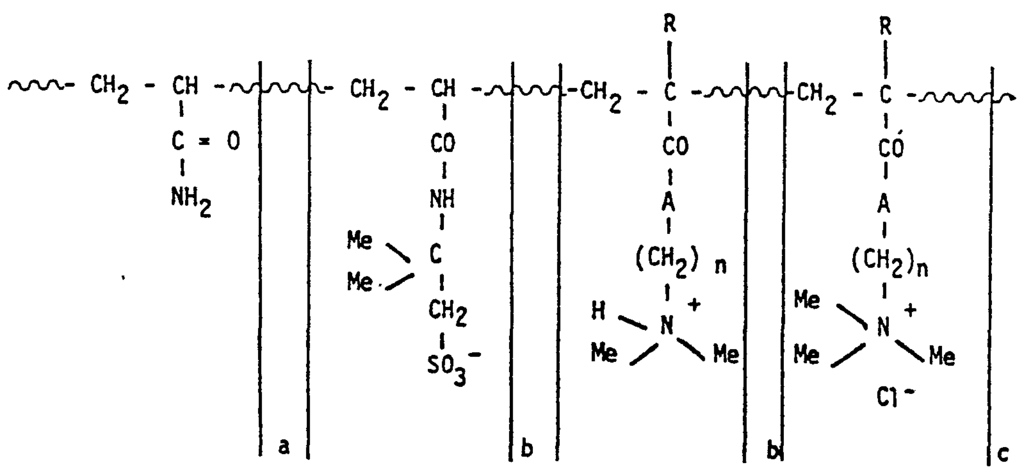

in which A represents an oxygen atom or an NH group, R represents a hydrogen atom or a methyl group when A represents an NH group and only a hydrogen atom when A represents an oxygen atom, *n* is equal to 2 when A represents an oxygen atom and *n* is equal to 3 when A represents an NH group, the letters *a, b,* and *c* represent the molar % of each monomer used and they have the following relationships:

$$a + 2b + c = 100$$

$$30 < a < 75$$

$$10 < b < 25$$

$$5 < c < 20$$

2. Water-soluble cationic ampholytic tetrapolymers according to claim 1, additionally characterized in that they result from the copolymerization of:

a moles of acrylamide, AAM;

b moles of 2-acrylamido-2-methylpropanesulphonic acid, AMPS;

b moles of a basic monomer chosen from amongst the following three monomers:

dimethylaminoethyl acrylate, DMAEA

N-(3-dimethylamino-1-propyl)acrylamide, DMAPA;

N-(3-dimethylamino-1-propyl)methacrylamide, DMAPMA;

c molesof a cationic monomer chosen from amongst the following four monomers:

N,N,N-trimethyl-3-acrylamidopropanaminium chloride, TMAPC;

N,N,N-trimethyl-3-methacrylamidopropanaminium chloride, TMMAPC;

N,N,N-trimethyl-2-acryloyloxyethanaminium chloride, MAC;

dimethylaminoethyl acrylate hydrochloride, AHC;

with the following relationships:

$$a + 2b + c = 100$$

$$30 < a < 75$$

$$10 < b < 25$$

$$5 < c < 20$$

3. Tetrapolymer according to claim 1 or 2, characterized in that it contains, in molar proportions, 75% of acrylamide, 10% of 2-acrylamido-2-methylpropanesulphonic acid, 10% of N-(3-dimethylamino-1-propyl)methacrylamide and 5% of N,N,N-trimethyl-3-methacrylamidopropanaminium chloride.

4. Tetrapolymer according to claim 1 or 2, characterized in that it contains, in molar proportions, 40% of acrylamide, 20% of 2-acrylamido-2-methylpropanesulphonic acid, 20% of N-(3-dimethylamino-1-propyl)-methacrylamide and 20% of N,N,N-trimethyl-3-methacrylamidopropanaminium chloride.

5. Use of the tetrapolymers according to any one of claims 1 to 4 in the treatment of keratinous fibres.

6. Aqueous compositions intended for the treatment of keratinous fibres, characterized in that they contain a tetrapolymer according to any one of claims 1 to 4 as the main active substances.

7. Aqueous compositions intended for hair care according to claim 6, characterized in that they contain from 0.1 to 5% by weight, expressed as dry matter, of a tetrapolymer according to any one of claims 1 to 4.

**Patentansprüche**

1. Ampholytische, kationische, wasserlösliche Tetrapolymere der folgenden allgemeinen Formel:

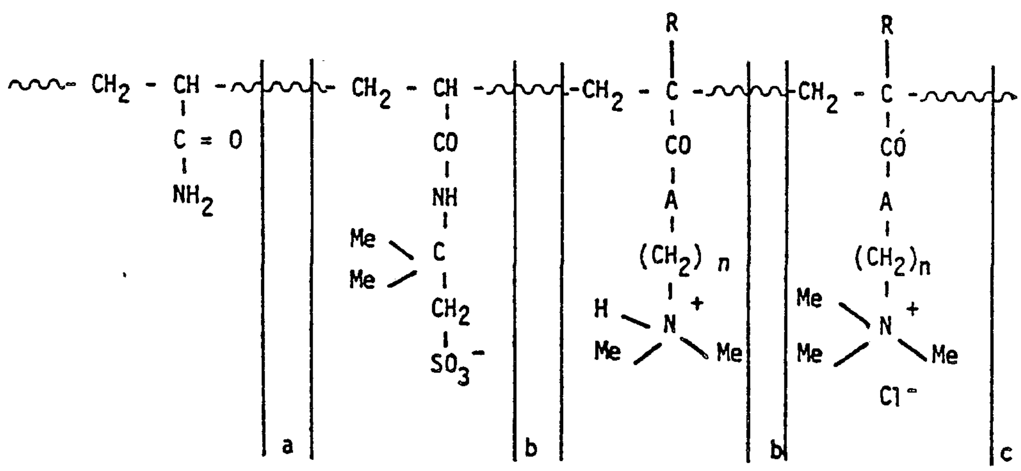

worin A ein Sauerstoffatom oder die Gruppe NH bedeutet, R ein Wasserstoffatom oder eine Methylgruppe, falls A eine Gruppe NH darstellt, und nur ein Wasserstoffatom, falls A ein Sauerstoffatom darstellt, bedeutet, n gleich 2 ist, wenn A ein Sauerstoffatom bedeutet, und n gleich 3 ist, wenn A eine Gruppe NH bedeutet, wobei die Buchstaben *a, b, c,* die Mol-% jedes verwendeten Monomeren bedeuten und diese den folgenden Beziehungen entsprechen:

$$a + 2b + c = 100$$

$$30 < a < 75$$

$$10 < b < 25$$

$$5 < c < 20$$

2. Amhpolytische, kationische, wasserlösliche Tetrapolymere nach Anspruch 1, dadurch gekennzeichnet, daß sie aus der Copolymerisation herrühren von:

a Mol Acrylamid, AAM;

b Mol 2-Acrylamido-2-methyl-propansulfonsäure, AMPS;

b Mol eines aus den drei folgenden Monomeren ausgewählten basischen Monomeren:
- Dimethylaminoethylacrylat, ADAME;
- N-(3-Dimethylamino-1-propyl)-acrylamid, DMAPA;
- N-(3-Dimethylamino-1-propyl)-methacrylamid, DMAPMA;

c Mol eines aus den folgenden vier Monomeren ausgewählten, kationischen Monomeren:
- N,N,N-3-Trimethylacrylamido-propanaminium, APTAC;
- N,N,N-3-Trimethylmethacrylamido-propanaminium, MAPTAC;
- N,N,N-2-Trimethylacryloyloxy-ethanaminium, CMA;
- Dimethylaminoethylacrylat-chlorhydrat, CHA;

mit folgenden Beziehungen:

$$a + 2b + c = 100$$

$$30 < a < 75$$

$$10 < b < 25$$

$$5 < c < 20$$

3. Tetrapolymeres nach Anspruch 1 oder 2, dadurch gekennzeichnet, daß es in Mol-Verhältnissen enthält: 75% Acrylamid, 10% 2-Acrylamido-2-methyl-propansulfonsäure, 10% N-(3-Dimethylamino-1-propyl)-methacrylamid und 5% N,N,N-3-Trimethylmethacrylamido-propanaminium.

4. Tetrapolymeres nach Anspruch 1 oder 2, dadurch gekennzeichnet, daß es in Mol-Verhältnissen enthält: 40% Acrylamid, 20% 2-Acrylamido-2-methyl-propansulfonsäure, 20% N-(3-Dimethylamino-1-propyl)-methacrylamid und 20% N,N,N-3-Trimethylmethacrylamido-propanaminium.

5. Anwendung der Tetrapolymere nach einem der Ansprüche 1 bis 4 zur Behandlung von Keratinfasern.

6. Wässrige Zusammensetzungen, bestimmt zur Behandlung von Keratinfasern, dadurch gekennzeichnet, daß sie als hauptsächliche Aktivmaterialien ein Tetrapolymeres nach einem der Ansprüche 1 bis 4 enthalten.

7. Wässrige, zur Haarpflege bestimmte Zusammensetzungen nach Anspruch 6, dadurch gekennzeichnet, daß sie gewichtsmäßig, ausgedrückt als Trockenmaterial 0,1 bis 5% eines Tetrapolymeren nach einem der Ansprüche 1 bis 4 enthalten.